# EUROPÄISCHE PATENTSCHRIFT

(11) **EP 1 208 071 B1**
(45) Veröffentlichungstag und Bekanntmachung des Hinweises auf die Patenterteilung: **11.08.2004**
(21) Anmeldenummer: 00964055.8
(22) Anmeldetag: 29.08.2000
(51) Int. Cl.: C07C 45/60, C07C 47/12

(54) **VERFAHREN ZUR KONTINUIERLICHEN HERSTELLUNG VON GLUTARALDEHYD**
METHOD FOR THE CONTINUOUS PRODUCTION OF GLUTARALDEHYDE
PROCEDE DE PRODUCTION EN CONTINU DE GLUTARALDEHYDE

(30) Priorität: 30.08.1999 DE 19941132
(43) Veröffentlichungstag der Anmeldung: 29.05.2002
(73) Patentinhaber: BASF AKTIENGESELLSCHAFT, 67056 Ludwigshafen (DE)
(72) Erfinder: THERRE, Jörg, 67551 Worms (DE); OOST, Carsten, 67098 Bad Dürkheim (DE)
(74) Vertreter: Kinzebach, Werner, Dr.
(86) Internationale Anmeldenummer: PCT/EP2000/008412
(87) Internationale Veröffentlichungsnummer: WO 2001/016237

(56) Entgegenhaltungen:
- EP-A- 0 697 392
- EP-A- 0 717 026
- WO-A-99/23088
- US-A- 2 546 018

## Beschreibung

Die Erfindung betrifft ein Verfahren zur kontinuierlichen Herstellung von Glutaraldehyd durch Umsetzung von Alkoxydihydropyranen der allgemeinen Formel I 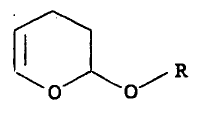 in der R für geradkettiges oder verzweigtes C₁-C₂₀-Alkyl, bevorzugt für C₁-C₈-Alkyl, und insbesondere für die Methylgruppe steht, mit Wasser bei einer Temperatur im Bereich von 0 °C bis 200 °C, vorzugsweise 40 bis 150 °C, und einem Druck im Bereich von 0,01 bar bis 16 bar, vorzugsweise 0,1 bis 5 bar.

Glutaraldehyd wird beispielsweise als Mikrobiozid oder in der Gerberei verwendet.

In der US 4,244,876 und der zugehörigen US 4,448,977 werden lagerfähige Zusammensetzungen aus Glutaraldehydacetalen und einer organischen Säure als Katalysator beschrieben, aus denen bei Bedarf durch Wasserzugabe rasch eine wäßrige Lösung von Glutaraldehyd hergestellt werden kann. Diese Lösung wird vor allem als Biozid zur Kontrolle von Schwefelbakterien in Ölquellen verwendet. Zu diesem Zweck ist eine Auftrennung des Reaktionsgemisches in seine reinen Komponenten nicht erforderlich sondern es wird das ungereinigte Hydrolyseprodukt verwendet. Es wird ein 2-Alkoxy-3,4-dihydropyran mit einem Alkohol oder Glycol unter Säurekatalyse, z.B. mit einem stark sauren Ionenaustauscher, zu einer Mischung von 2,6-Dialkoxytetrahydropyran, Dialkoxypentanal und 1,1,5,5-Tetraalkoxypentan umgesetzt. Nach Abtrennen des Ionenaustauschers wird eine organische Säure zur Mischung zugegeben. Die so erhaltene Mischung ist lagerfähig. Aus ihr kann am Einsatzort, z.B. einer Bohrstelle, durch Wasserzugabe eine glutaraldehydhaltige wäßrige Lösung hergestellt werden.

In der europäischen Patentanmeldung EP 0 066 224 Al wird ein Verfahren zur Herstellung einer im wesentlichen wasserfreien Glutaraldehydvorstufe beschrieben, aus der bei Wasserzugabe innerhalb kurzer Zeit Glutaraldehyd freigesetzt werden kann. Zur Herstellung der lagerfähigen Vorstufe wird ein 2-Alkoxy-3,4-dihydropyran in Gegenwart eines sauren Katalysators, z.B. eines sauren Ionenaustauschers, bei einer Temperatur von 30 bis ungefähr 100 °C mit Wasser in einem Molverhältnis von 1 : 1 bis 3 : 1 umgesetzt. Es ergibt sich ein Gemisch, das als Hauptkomponente 2-Hydroxy-6-methoxytetrahydropyran neben Spuren von 2,6-Dimethoxytetrahydropyran und Glutaraldehyd enthält. Nach Abtrennung des sauren Katalysators, z.B. durch Filtration, ist das Gemisch lagerfähig. Bei Wasserzugabe erfolgt Hydrolyse unter Bildung von Glutaraldehyd. Eine Reinigung des Glutaraldehyds ist nicht vorgesehen.

In der JP 72 26 488 wird ein Verfahren zur kontinuierlichen Herstellung von Glutaraldehyd durch Hydrolyse eines 2-Alkoxy-3,4-dihydropyrans unter Säurekatalyse bekannt. Dazu wird ein Gemisch des 2-Alkoxy-3,4-dihydropyrans und Wasser in einem Molverhältnis zwischen 1 : 2 bis 1 : 100 auf 50 bis 200 °C erwärmt. Der Prozeß wird so geführt, daß eine Konzentration von 0,5 bis 10 Gew.-% des 2-Alkoxy-3,4-dihydropyrans im System eingehalten wird. Eine weitere Reinigung ist nicht vorgesehen. Das erhaltene Reaktionsprodukt wird als Gerbmittel für Leder verwendet.

Die US 2,546,018 beschreibt die Synthese von Glutaraldehyd und C-substituierten Glutaraldehyden ausgehend von Dihydropyranen. Die Hydrolyse des Dihydropyrans kann mit oder ohne Säurekatalyse durchgeführt werden, wobei eine unkatalysierte Reaktion wegen der einfacheren Aufarbeitung des abreagierten Gemisches bevorzugt ist. Die Reaktion kann batchweise durchgeführt werden, wobei auch vorgeschlagen wird, den entstehenden Alkohol kontinuierlich während der Reaktion aus dem Reaktionsgemisch zu entfernen. Weiter wird auch vorgeschlagen, die Reaktion kontinuierlich durchzuführen. Dazu soll das Reaktionsgemisch durch einen rohrförmigen Reaktor geführt werden, der auf eine geeignete Temperatur erwärmt wird. Die Fließrate wird dabei so gewählt, daß am Ausgang des Reaktors die erwünschte Umsetzung stattgefunden hat. Nachdem das Reaktionsgemisch den Reaktor verlassen hat, kann dies entweder zunächst gesammelt und dann batchweise destilliert werden oder es wird alternativ eine kontinuierliche Destillation mit Abtrennung der gewünschten Fraktionen vorgeschlagen. In den Beispielen der Patentschrift wird jeweils in einem ersten Schritt die Hydrolyse des Dihydropyrans und anschließend in einem zweiten Schritt eine fraktionierte Destillation durchgeführt. Nachteilig an diesem Verfahren ist, daß eine umfangreiche Apparatur erforderlich ist, wobei in einer ersten Stufe mit einer ersten Apparatur die Hydrolyse durchgeführt wird und in einer zweiten Stufe mit einer zweiten Apparatur das erwünschte Produkt durch fraktionierte Destillation abgetrennt wird. Dies ist umständlich und verursacht Kosten.

Die EP-A 0 697 392 und die EP-A 0 717 026 beschreiben Verfahren zur Herstellung von Glutardialdehyd durch Umsetzung von Alkoxydihydropyranen mit Wasser, wobei als Katalysatoren Bleicherden bzw. mikroporöse, kristalline Aluminiumsilikate verwendet werden.

Aufgabe der Erfindung ist daher, ein Verfahren zur Verfügung zu stellen, mit dem auf einfache Weise kontinuierlich Glutaraldehyd oder C-substituierte Glutaraldehyde mit erhöhter Reinheit hergestellt werden können, wobei der apparative Aufwand möglichst gering sein soll, um die Produktionskosten niedrig zu halten.

Die Aufgabe wird gelöst durch ein Verfahren zur kontinuierlichen Herstellung von Glutaraldehyd durch Umsetzung eines Alkoxydihydropropans der Formel I bei einer Temperatur im Bereich von 0 °C bis 200 °C und einem Druck im Bereich von 0,01 bar bis 16 bar unter Bildung von Glutaraldehyd und dem der Alkoxygruppe entsprechenden Alkohol, wobei Wasser und Alkoxydihydropyran kontinuierlich einer Reaktionskolonne zugeführt werden, vorzugsweise an einer Stelle zwischen Kopf und Sumpfablauf der Reaktionskolonne, und am Kopf der Kolonne ein mit dem der Alkoxygruppe entsprechenden Alkohol angereichertes Destillat und am Sumpf ein mit Glutaraldehyd angereichertes Produkt abgenommen wird. Die Kolonne weist Packungen und/oder Einbauten auf, die ausgewählt sind unter Füllkörpern, strukturierten Packungen aus Blech oder Metallgewebe, Böden oder katalytisch aktiven Packungen fester Säuren, wobei die Kolonne 2 bis 200 theoretische Trennstufen aufweist und ein Teil der theoretischen Trennstufen zwischen der Zugabestelle des Alkoxydihydropyrans und dem Sumpf der Kolonne und ein Teil der theoretischen Trennstufen zwischen der Zugabestelle des Alkoxydihydropyrans und dem Kopf der Kolonne liegt.

Die für die Reaktion verwendete Vorrichtung kann sehr kompakt gestaltet werden, da die Hydrolyse des Alkoxydihydropyrans in der Kolonne durchgeführt wird und somit auf ein separates Gefäß für die Durchführung der Hydrolysereaktion verzichtet werden kann. Durch die kontinuierliche Reaktionsführung wird die Dimension der Anlage bei gleichem Umsatz pro Zeit im Vergleich zu einer diskontinuierlichen Reaktionsführung mit separatem Abdestillieren des gebildeten Alkohols bzw. des Glutaraldehyds wesentlich verringert. Durch die Wahl geeigneter Reaktionsparameter bzw. geeigneter Dimensionierung der Anlage kann ein nahezu vollständiger Umsatz des Alkoxydihydropyrans erreicht werden, so daß eine erhöhte Ausbeute erzielt werden kann, wobei weiter der gebildete Glutaraldehyd eine im Vergleich zu bisherigen Verfahren höhere Reinheit aufweist.

Die Reaktionsgeschwindigkeit kann erhöht werden, indem die Umsetzung unter Säurekatalyse durchgeführt wird. Geeignete Säuren sind organische Säuren, wie z.B. gesättigte und ungesättigte Carbonsäuren mit 1 bis 10 Kohlenstoffatomen. Es können auch polyfunktionelle Säuren, wie z.B. Maleinsäure verwendet werden. Bevorzugt ist die Verwendung anorganischer Säuren, wie z.B. Phosphorsäure, Borsäure, Salpetersäure, Schwefelsäure oder saurer Salze, wie z.B. NaH₂PO₄. Wenn unter den Betriebsbedingungen der Reaktionskolonne schwerflüchtige Säuren verwendet werden, werden diese bevorzugt im oberen Teil der Reaktionskolonne zugegeben. Bei der Verwendung von unter den Betriebsbedingungen flüchtigen Säuren können diese auch im unteren oder mittleren Teil der Reaktionskolonne zugegeben werden. Die Menge an Säure ist so zu bemessen, daß im Sumpfablauf der Kolonne eine Säurekonzentration zwischen 0,0001 Gew.-% und 10 Gew.-%, insbesondere zwischen 0,01 Gew.-% und 3 Gew.-% erreicht wird. Die Katalysatoren werden bevorzugt gelöst eingesetzt. Als Lösungsmittel sind Wasser, Alkohol, das Alkoxydihydropyran oder Glutaraldehyd-Wasser-Mischungen geeignet.

Anstelle flüssiger Säuren können auch katalytisch aktive Packungen fester Säuren in der Reaktionskolonne angeordnet sein. Solche festen Säuren sind z.B. Ionenaustauscher, wie z.B. Amberlyst® 15 oder die in DE 44 29 262 genannten Bleicherdekatalysatoren. Die Katalysatoren sind in der Kolonne im Allgemeinen so angeordnet, daß ein intensiver Kontakt der Reaktionslösung mit dem Katalysator möglich ist, der feste Katalysator aber durch Siebe oder Filter in der Kolonne festgehalten wird. Ein Beispiel einer solchen Anordnung ist die KATAPAK® der Firma Sulzer. Als feste Katalysatoren können auch Dünnschichtkatalysatoren verwendet werden, bei denen eine katalytisch aktive Masse direkt auf Einbauten, Packungen oder Füllkörper der Kolonne, z.B. durch Tränken und anschließendem Trocknen aufgebracht ist.

Die Zahl der theoretischen Trennstufen liegt im Bereich von 2 bis 200, vorzugsweise 3 bis 100, insbesondere 4 bis 50.

Die Abtrennung des Alkohols wird weiter verbessert, wenn die Reaktionskolonne unter Rücklauf betrieben wird und das Rücklaufverhältnis zwischen 0,2 und 80, vorzugsweise zwischen 0,4 und 40 gewählt wird.

Wird am Kopf der Reaktionskolonne der Alkohol als Azeotrop mit Wasser abdestilliert, ist es vorteilhaft, wenn mit dem Kopf der Reaktionskolonne ein Phasentrenngefäß verbunden ist und eine sich abscheidende wäßrige Phase auf den Kopf der Reaktionskolonne zurückgeführt wird. In diesem Fall kann gegebenenfalls auf einen zusätzlichen Rücklauf auf die Kolonne verzichtet werden.

Die Heizleistung im Sumpf der Kolonne wird vorzugsweise so gewählt, daß der Sumpfablauf der Kolonne weniger als 10 Gew.-%, vorzugsweise weniger als 1 Gew.-% des bei der Hydrolysereaktion freiwerdenden Alkohols enthält.

Die Konzentration des Glutaraldehyds im Sumpfablauf beträgt 5 bis 75 Gew.-%, vorzugsweise 25 bis 65 Gew.-%.

Wasser und Alkoxydihydropyran können voneinander getrennt oder im Gemisch in die Reaktionskolonne geführt werden. Bei getrennter Zufuhr wird das Wasser bevorzugt im oberen Teil der Reaktionskolonne zugegeben. Das Alkoxydihydropyran wird bevorzugt im unteren Teil der Reaktionskolonne zugegeben. Zwischen der Zugabestelle des Alkoxydihydropyrans und dem Sumpf der Reaktionskolonne bzw. dem Kopf der Kolonne sollen so viele theoretische Trennstufen liegen, daß ein spezifikationsgerechter Gehalt des Alkoxydihydropyrans im Sumpfprodukt bzw. Kopfprodukt erreicht wird. Diese Zahl der erforderlichen Stufen ist vom Rücklaufverhältnis abhängig und kann durch Vorversuche leicht ermittelt werden. Bei Zufuhr als Gemisch werden Wasser und Alkoxydihydropyran im Allgemeinen im mittleren Bereich der Reaktionskolonne eingespeist.

Die Zugabemenge des Alkoxydihydropyrans ist vom Volumen der verwendeten Reaktionskolonne, von der Menge und der Art des Katalysators und von der Temperatur in der Reaktionskolonne abhängig und kann durch Vorversuche leicht ermittelt werden. Im Allgemeinen können pro 1 m³ Leervolumen der Kolonne 1000 bis 5000 kg/h Alkoxydihydropyran zugegeben werden.

Die Menge an Wasser ist dabei so zu bemessen, daß am Sumpf der Reaktionskolonne der Glutaraldehyd mit der gewünschten Konzentration erhalten wird. Im Allgemeinen aber ist das Molverhältnis von Wasser zu Alkoxydihydropyran ≥ 3, insbesondere ≥ 4.

Die Reaktionskolonne kann verschiedene Packungen bzw. Einbauten aufweisen, nämlich Füllkörper (z.B. Prallringe), strukturierte Packungen aus Blech (z.B. Sulzer Mellapak 250Y) oder Metallgewebe (z.B. Sulzer BX oder CY) oder bevorzugt Böden (z.B. Glockenböden, Ventilböden, Tunnelböden) oder auch Kombinationen der genannten Einbauten. Gegebenenfalls können auch spezielle Verweilzeitböden mit einem besonders hohen Flüssigkeitsstand oder spezielle Konstruktionen der Ablaufschächte mit besonders großem Flüssigkeitsinhalt verwendet werden. Ferner können auch Packungen fester Säuren, wie Ionenaustauscher oder Bleicherdekatalysatoren in der Reaktionskolonne angeordnet sein. Diese Packungen sind so gestaltet, daß ein intensiver Kontakt der Reaktionslösung mit dem Katalysator möglich ist, der feste Katalysator aber durch Siebe oder Filter in der Kolonne festgehalten wird. Ein Beispiel einer solchen Anordnung ist die KATAPAK der Firma Sulzer.

Die Dimensionierung der Reaktionskolonne kann verringert werden, wenn im mittleren Teil der Reaktionskolonne ein Seitenabzug zur Entnahme von gasförmigen oder vorzugsweise flüssigem Produkt, das Wasser, Alkoxydihydropyran und gegebenenfalls den Katalysator umfasst, entnommen und in einen angeschlossenen Reaktionsraum überführt wird, wobei der Ablauf aus dem Reaktionsraum in die Reaktionskolonne, vorzugsweise im mittleren Bereich der Kolonne und unterhalb des Seitenabzugs, zurückgeführt wird. Der angeschlossene Reaktionsraum kann als Rührkessel, Schlaufenreaktor oder Rohrreaktor ausgebildet sein.

Die Menge des Seitenabzuges kann in einem weiten Bereich variieren. Vorzugsweise entspricht sie dem 0,1- bis 80-fachen, vorzugsweise dem 0,2- bis 30-fachen der der Reaktionskolonne zugeführten Alkoxydihydropyranmenge.

Bevorzugt werden das Wasser, das Alkoxydihydropyran und ggf. der Katalysator dem angeschlossenen Reaktionsraum zugeführt.

Die Erfindung wird im weiteren durch Beispiele und unter Bezugnahme auf die beigefügte Zeichnung genauer erläutert, ohne sie zu begrenzen. Gleiche Teile sind dabei mit denselben Bezugsziffern bezeichnet. Es zeigen:
- Fig.1:: eine schematische Darstellung einer ersten Ausführungsform einer Vorrichtung zur Durchführung des erfindungsgemäßen Verfahrens;
- Fig.2:: eine zweite Ausführungsform einer Vorrichtung zur Durchführung des erfindungsgemäßen Verfahrens.

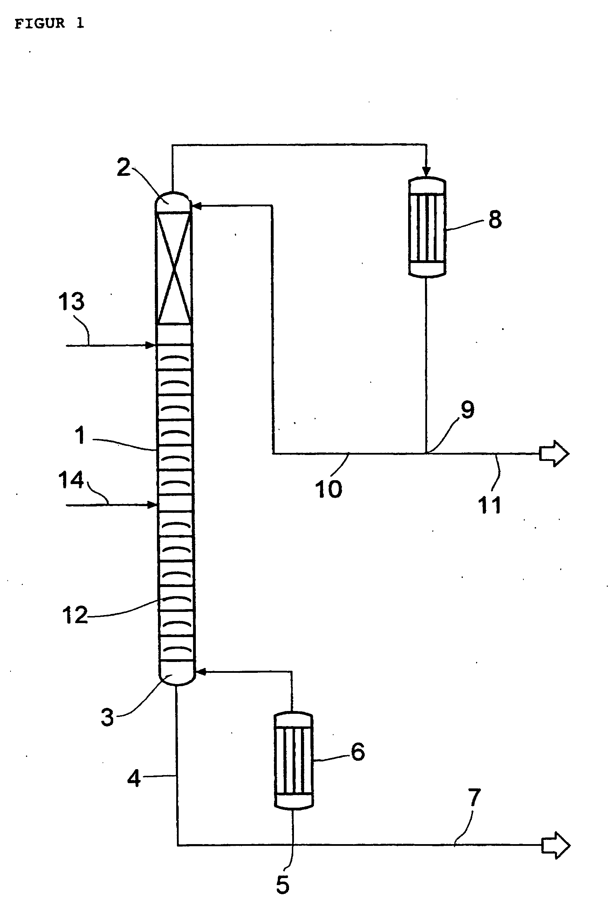

Die in Figur 1 gezeigte Vorrichtung umfaßt eine Reaktionskolonne 1 mit einem Kopf 2 und einem Sumpf 3. Die Kolonne ist mit einer der oben beschriebenen Packungen ausgestattet. Der Sumpfablauf wird über Leitung 4 zu einer Verzweigung 5 geführt, wo ein Teil des Ablaufs in einem Verdampfer 6 erneut verdampft und in den unteren Teil der Kolonne 1 zurückgeführt wird. Ein Teil des aus Glutaraldehyd in Wasser bestehenden Ablaufs wird über Leitung 7 abgenommen. Am Kopf 2 der Reaktionskolonne 1 wird das Destillat einem Kondensator 8 zugeführt und kondensiert. Ein Teil des Kondensats wird über die Verzweigung 9 als Rücklauf über die Leitung 10 wieder auf den Kopf 2 der Reaktionskolonne 1 zurückgeführt und ein Teil des Alkohols wird über Leitung 11 ausgeschleust.

Das Wasser wird über Leitung 13, ggf. im Gemisch mit Katalysator, dem oberen Teil der Reaktionskolonne 1 zugeführt. Die zugeführte Wassermenge ergibt sich aus dem gewünschten Glutaraldehydgehalt der über Leitung 7 abgenommenen wäßrigen Lösung.

Das Alkoxydihydropyran wird über Leitung 14 der Reaktionskolonne 1 zugeführt. Die Anordnung der Leitung 14 ist dabei so gewählt, daß zwischen der Zugabestelle des Alkoxydihydropyrans und dem Sumpf der Reaktionskolonne so viele theoretische Trennstufen liegen, daß ein spezifikationsgerechter Gehalt des Alkoxydihydropyrans im Sumpfprodukt erhalten wird. Zwischen der Zugabestelle des Alkoxydihydropyrans und dem Kopf 3 der Reaktionskolonne 1 sollen so viele theoretische Trennstufen liegen, daß ein spezifikationsgerechter Gehalt des Alkoxydihydropyrans im erzeugten Alkohol erreicht wird.

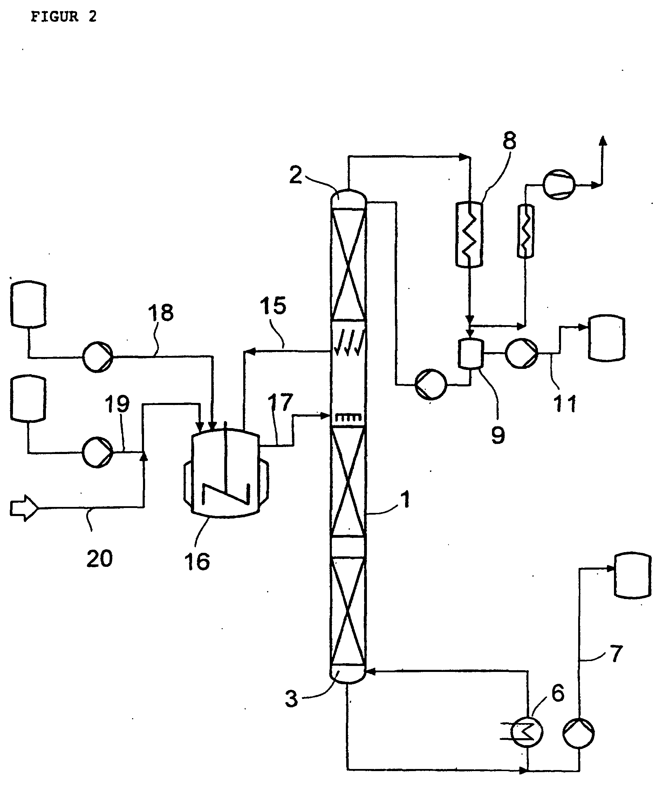

Figur 2 zeigt eine zweite Ausführungsform einer Vorrichtung zur Durchführung des erfindungsgemäßen Verfahrens. Wie bei der in Figur 1 dargestellten Vorrichtung ist eine Reaktionskolonne 1 vorgesehen, an deren Kopf 2 Destillat abgenommen und einem Kondensator 8 zugeführt wird. Das Kondensat wird teilweise dem Kopf 2 als Rücklauf über die Verzweigung 9 wieder zugeführt und zum Teil über Leitung 11 ausgeschleust. Am Sumpf 3 der Reaktionskolonne 1 wird ein Teil des Sumpfablaufs durch Verdampfer 6 wieder verdampft, bzw. über Leitung7dem System entnommen.

Über Leitung 15 wird der Reaktionskolonne 1 ein gasförmiger oder bevorzugt flüssiger Seitenabzug entnommen und in den außerhalb der Reaktionskolonne 1 angeordneten Behälter 16 geführt. Der Behälter 16 ist hier als Rührkessel ausgeführt, er kann jedoch auch z.B. als Schlaufen- oder Rohrreaktor ausgeführt sein. Der Ablauf aus dem Kessel 16 wird über Leitung 17 wieder der Reaktionskolonne 1 zugeführt. Über Leitungen 18 und 19 werden dem Kessel 16 das Alkoxydihydropyran und das Wasser zugeführt, wobei in die Wasserzuführung 19 die Leitung 20 für die Zuführung des flüssigen Katalysators mündet.

### Beispiel 1

In einer Vorrichtung gemäß Figur 1 wurde Methoxydihydropyran (MOP) mit Wasser in einer kontinuierlichen Reaktivdestillation zu Glutaraldehyd und Methanol verseift. Die Reaktionskolonne bestand aus einer Bodenkolonne mit 20 praktischen Böden und einem Innendurchmesser von 50 mm, die bei 400 mbar betrieben wurde. Auf den vierten Boden von oben wurden 132 g/h einer 0,6 gewichtsprozentigen Lösung von Maleinsäure in Wasser und auf den dreizehnten Boden von oben wurden 150 g/h MOP gegeben. Durch einen elektronischen Rückflußteiler mit Siedegrenze wurde die Kopftemperatur durch Veränderung des Rücklaufverhältnisses auf 44 °C gehalten. Die Heizleistung im Umlaufverdampfer 6 wurde so geregelt, daß im Sumpf 78 °C erreicht wurden. Der Sumpf enthielt 44 Gew.-% Wasser, 1,3 Gew.-% Methanol und 54 Gew.-% Glutaraldehyd. Im Destillat waren 85 Gew.-% Methanol, 1,5 Gew.-% Wasser und 13,5 Gew.-% MOP enthalten.

### Beispiel 2

In der im Beispiel 1 beschriebenen und bei 400 mbar betriebenen Vorrichtung wurden auf den sechsten Boden von oben 120 g/h einer 0,6 gew.-%igen Lösung von Maleinsäureanhydrid in Wasser und auf den zwölften Boden von oben wurden 147 g/h MOP gegeben. Durch einen elektronischen Rückflußteiler wurde ein Rückflußverhältnis von 5 : 1 eingestellt. Die Heizleistung im Umlaufverdampfer 6 wurde so geregelt, daß im Sumpf 75 °C erreicht wurden. Der Sumpf enthielt 43,85 Gew.-% Wasser, 2,82 Gew.-% Methanol und 53,33 Gew.-% Glutaraldehyd. Das Destillat enthielt 90,65 Gew.-% Methanol, 1,16 Gew.-% Wasser und 9,09 Gew.-% MOP.

### Beispiel 3

Eine Vorrichtung gemäß Figur 1 war mit einer Reaktionskolonne ausgestattet, die aus einer Glockenbodenkolonne mit einem Innendurchmesser von 50 mm mit 20 praktischen Böden bestand, auf die ein Kolonnenschuß mit 50 cm Sulzer DX Packung aufgesetzt war. Der Druck im Sumpf der Kolonne betrug 640 mbar, der Druck am Kopf der Kolonne betrug 595 mbar. Auf den Kopf der Kolonne wurden 1380 ml/h Destillat als Rücklauf gepumpt. Auf den obersten Boden der Kolonne wurden 130 g/h einer 0,6 gewichtsprozentigen Lösung von Phosphorsäure in Wasser und auf den dreizehnten Boden von oben der Glockenbodenkolonne wurdem 150 g/h MOP gegeben. Die Heizleistung im Verdampfer wurde so geregelt, daß im Sumpf 89 °C erreicht wurden. Der Sumpf enthielt 48 Gew.-% Wasser, 0,6 Gew.-% Methanol, 0,5 Gew.-% MOP und 49 Gew.-% Glutaraldehyd. Im Destillat waren 96 Gew.-% Methanol und 1,7 Gew.-% MOP enthalten.

### Beispiel 4

Eine Vorrichtung gemäß Figur 2 umfaßte als Reaktionskolonne eine Packungskolonne, die im unteren Teil mit 96 cm Sulzer-CY-Packung und im oberen Teil mit 50 cm Sulzer-DX-Packung gefüllt war. Zwischen den beiden Kolonnenschüssen befand sich ein flüssiger Seitenabzug. Der Zulauf zur Kolonne befand sich 11 cm oberhalb des Seitenabzuges. Der Innendurchmesser der Kolonne war 50 mm. Der Druck im Sumpf der Kolonne betrug 619 mbar, der Druck am Kopf der Kolonne betrug 600 mbar. Das Rücklaufverhältnis am Kopf der Kolonne war 17,3. Am Seitenabzug der Kolonne wurden 2440 ml/h entnommen und in einen von außen mit Öl auf 74 °C beheizten Reaktor gegeben. Der Reaktor wurde durch Rühren und Umwälzen von 1040 ml/h durchmischt. Die Masse der Reaktionsmischung im Reaktor war 900 g. In den Reaktor wurden 150 g/h MOP und 130 g/h Wasser gegeben. Im Wasserwaren 0,18 Gew.-% Phosphorsäure gelöst. Am Kopf der Kolonne wurden 45 g/h Destillat und am Sumpf 235 g/h Produkt entnommen. Die Heizleistung im Verdampfer wurde so geregelt, daß im Sumpf 87 °C erreicht wurden. Das Destillat enthielt 99,05 Gew.-% Methanol und 0,95 Gew.-% MOP. Im Sumpf waren 47,3 Gew.-% Wasser und 51 Gew.-% Glutaraldehyd enthalten. Das Sumpfprodukt enthielt weder Methanol noch MOP.

## Patentansprüche

1. Verfahren zur kontinuierlichen Herstellung von Glutaraldehyd durch Umsetzung eines Alkoxydihydropyrans der allgemeinen Formel I 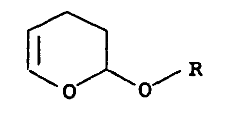 in der R für C₁-C₂₀-Alkyl steht, mit Wasser bei einer Temperatur im Bereich von 0 °C bis 200 °C und einem Druck im Bereich von 0,01 bar bis 16 bar unter Bildung von Glutaraldehyd und dem der Alkoxygruppe entsprechenden Alkohol, **dadurch gekennzeichnet, dass** Wasser und Alkoxydihydropyran kontinuierlich einer Reaktionskolonne an einer Stelle zwischen Kopf und Sumpfablauf der Kolonne zugeführt werden, wobei die Kolonne Packungen und/oder Einbauten aufweist, die ausgewählt sind unter Füllkörpern, strukturierten Packungen aus Blech oder Metallgewebe, Böden oder katalytisch aktiven Packungen fester Säuren, wobei die Kolonne 2 bis 200 theoretische Trennstufen aufweist und ein Teil der theoretischen Trennstufen zwischen der Zugabestelle des Alkoxydihydropyrans und dem Sumpf der Kolonne und ein Teil der theoretischen Trennstufen zwischen der Zugabestelle des Alkoxydihydropyrans und dem Kopf der Kolonne liegt, und am Kopf der Kolonne ein mit dem der Alkoxygruppe entsprechenden Alkohol angereichertes Destillat und am Sumpf ein mit Glutaraldehyd angereichertes Produkt abgenommen wird.

2. Verfahren nach Anspruch 1, **dadurch gekennzeichnet, dass** die Umsetzung unter Säurekatalyse durchgeführt wird.

3. Verfahren nach Anspruch 1 oder 2, **dadurch gekennzeichnet, dass** in der Reaktionskolonne katalytisch aktive Packungen fester Säuren angeordnet sind.

4. Verfahren nach einem der vorhergehenden Ansprüche, **dadurch gekennzeichnet, dass** die Säuremenge so bemessen wird, dass im Sumpfablauf der Reaktionskolonne eine Säurekonzentration zwischen 0,0001 Gew.-% und 10 Gew.-%, insbesondere zwischen 0,01 Gew.-% und 3 Gew.-%, erreicht wird.

5. Verfahren nach einem der vorhergehenden Ansprüche, **dadurch gekennzeichnet, dass** am Kopf der Reaktionskolonne ein Rücklaufverhältnis im Bereich von 0,2 bis 80, vorzugsweise 0,4 bis 40, eingestellt wird.

6. Verfahren nach einem der vorhergehenden Ansprüche, **dadurch gekennzeichnet, dass** die Heizleistung im Sumpf der Reaktionskolonne so gewählt wird, dass der Sumpfablauf der Kolonne weniger als 10 Gew.-%, vorzugsweise weniger als 1 Gew.-%, Alkohol enthält.

7. Verfahren nach einem der vorhergehenden Ansprüche, **dadurch gekennzeichnet, dass** die Konzentration des Glutaraldehyds im Sumpfablauf 5 bis 75 Gew.-%, vorzugsweise 26 bis 65 Gew.-%, beträgt.

8. Verfahren nach einem der vorhergehenden Ansprüche, **dadurch gekennzeichnet, dass** die Zuführung des Wassers und des Alkoxydihydropyrans in die Reaktionskolonne getrennt voneinander erfolgt.

9. Verfahren nach einem der vorhergehenden Ansprüche, **dadurch gekennzeichnet, dass** im mittleren Teil der Reaktionskolonne ein Seitenabzug entnommen und in einen Reaktionsraum überführt wird, wobei der Ablauf aus dem Reaktionsraum in die Reaktionskolonne zurückgeführt wird.

10. Verfahren nach Anspruch 9, **dadurch gekennzeichnet, dass** die Menge des Seitenabzugs dem 0,1- bis 80-fachen der der Reaktionskolonne zugeführten Alkoxydihydropyranmenge entspricht.

## Claims

1. A process for the continuous preparation of glutaraldehyde by reaction of an alkoxydihydropyran of the formula I 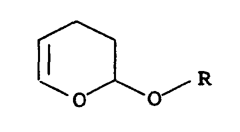 where R is C₁-C₂₀-alkyl, with water at from 0°C to 200°C and a pressure in the range from 0.01 bar to 16 bar to form glutaraldehyde and the alcohol corresponding to the alkoxy group, **characterized in that** water and alkoxydihydropyran are fed continuously to a reaction column at a point between the top and the bottom oulet of the column, wherein the column has packings and/or internals selected from random packing elements, structured packings made of sheetmetal or metal mesh, trays or catalytically active packings of solid acids, wherein the column has 2 to 200 theoretical plates and a portion of the theoretical plates is between the point of introduction of the alkoxydihydropyran and the bottom of the column and a portion of the theoretical plates is between the point of introduction of the alkoxydihydropyran and the top of the column and a distillate enriched in the alcohol corresponding to the alkoxy group is taken off at the top of the column and a product enriched in glutaraldehyde is taken off at the bottom.

2. A process as claimed in claim 1, **characterized in that** the reaction is carried out in the presence of an acid catalyst.

3. A process as claimed in claim 1 or 2, **characterized in that** catalytically active packing comprising solid acids is installed in the reaction column.

4. A process as claimed in any of the preceding claims, **characterized in that** the amount of acid is such that an acid concentration in the range from 0.0001% by weight to 10% by weight, in particular from 0.01% by weight to 3% by weight, is obtained in the stream leaving the bottom of the reaction column.

5. A process as claimed in any of the preceding claims, **characterized in that** a reflux ratio in the range from 0.2 to 80, preferably from 0.4 to 40, is set at the top of the reaction column.

6. A process as claimed in any of the preceding claims, **characterized in that** the heating power at the bottom of the reaction column is chosen so that the stream leaving the bottom of the column contains less than 10% by weight, preferably less than 1% by weight, of alcohol.

7. A process as claimed in any of the preceding claims, **characterized in that** the concentration of glutaraldehyde in the stream leaving the bottom of the column is from 5 to 75% by weight, preferably from 26 to 65% by weight.

8. A process as claimed in any of the preceding claims, **characterized in that** the water and the alkoxydihydropyran are fed separately into the reaction column.

9. A process as claimed in any of the preceding claims, **characterized in that** a stream is taken off at a side offtake in the middle section of the reaction column and is conveyed to a reaction space, and the stream flowing out of the reaction space is recirculated to the reaction column.

10. A process as claimed in claim 9, **characterized in that** the amount of material taken off at the side offtake corresponds to from 0.1 to 80 times the amount of alkoxydihydropyran fed to the reaction column.

## Revendications

1. Procédé de production continue de glutaraldéhyde par réaction d'un alkoxydihydropyranne de formule générale I 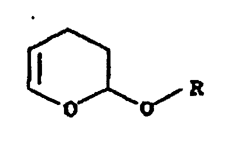 dans laquelle R représente un résidu alkyle en C₁-C₂₀, avec de l'eau à une température dans le domaine de 0°C à 200°C et à une pression dans le domaine de 0,01 bar à 16 bars, sous formation de glutaraldéhyde et de l'alcool correspondant au groupement alkoxy, **caractérisé en ce que** l'eau et l'alkoxydihydropyranne sont acheminés en continu à une colonne de réaction en un emplacement entre la tête et la décharge de puits de la colonne, la colonne présentant des garnitures et/ou des chicanes, qui sont sélectionnées parmi les corps de remplissage, les garnitures structurées en tôle ou des tissus métalliques, des plateaux ou des garnitures actives du point de vue catalytique d'acides solides, la colonne présentant de 2 à 200 étages de partition théoriques, et une partie des étages de partition théoriques se situant entre l'emplacement d'addition de l'alkoxydihydropyranne et le puits de la colonne, et une partie des étages de partition théoriques se situant entre l'emplacement d'addition de l'alkoxydihydropyranne et la tête de la colonne, et un distillat enrichi à l'alcool correspondant au groupement alkoxy étant prélevé à la tête de la colonne et un produit enrichi en glutaraldéhyde étant prélevé au puits de la colonne.

2. Procédé selon la revendication 1, **caractérisé en ce que** la réaction est effectuée sous catalyse acide.

3. Procédé selon la revendication 1 ou 2, **caractérisé en ce que** l'on dispose, dans la colonne de réaction, des garnitures actives du point de vue catalytique d'acides solides.

4. Procédé selon l'une quelconque des revendications précédentes, **caractérisé en ce que** la quantité d'acide est mesurée de telle sorte que l'on parvient, dans la décharge de puits de la colonne de réaction, à une concentration d'acide comprise entre 0,0001 % en poids et 10 % en poids, en particulier entre 0,01 % en poids et 3 % en poids.

5. Procédé selon l'une quelconque des revendications précédentes, **caractérisé en ce que** l'on ajuste, à la tête de la colonne de réaction, un rapport de reflux dans le domaine de 0,2 à 80, de préférence de 0,4 à 40.

6. Procédé selon l'une quelconque des revendications précédentes, **caractérisé en ce que** la puissance de chauffage dans le puits de la colonne de réaction est sélectionnée de telle sorte que la décharge de puits de la colonne contient moins de 10 % en poids, de préférence moins de 1 % en poids d'alcool.

7. Procédé selon l'une quelconque des revendications précédentes, **caractérisé en ce que** la concentration du glutaraldéhyde dans la décharge de puits est de 5 à 75 % en poids, de préférence de 26 à 65 % en poids.

8. Procédé selon l'une quelconque des revendications précédentes, **caractérisé en ce que** l'apport de l'eau et de l'alkoxydihydropyranne dans la colonne de réaction se fait d'une manière séparée l'un de l'autre.

9. Procédé selon l'une quelconque des revendications précédentes, **caractérisé en ce que**, dans la partie médiane de la colonne de réaction, un prélèvement latéral est extrait et acheminé dans un espace de réaction, la décharge hors de l'espace de réaction étant reconduite dans la colonne de réaction.

10. Procédé selon la revendication 9, **caractérisé en ce que** la quantité du prélèvement latéral correspond à un multiple allant de 0,1 à 80 fois la quantité d'alkoxydihydropyranne alimentée dans la colonne de réaction.
